(19) Europäisches Patentamt
European Patent Office
Office européen des brevets

(11) Veröffentlichungsnummer: **0 170 203**
**A2**

(12) # EUROPÄISCHE PATENTANMELDUNG

(21) Anmeldenummer: **85109282.5**

(22) Anmeldetag: **24.07.85**

(51) Int. Cl.[4]: **C 07 D 498/04**
A 01 N 43/90
//(C07D498/04, 265:00, 221:00)

(30) Priorität: **02.08.84 DE 3428476**

(43) Veröffentlichungstag der Anmeldung:
**05.02.86 Patentblatt 86/6**

(84) Benannte Vertragsstaaten:
**BE CH DE FR GB IT LI NL**

(71) Anmelder: **BASF Aktiengesellschaft**
**Carl-Bosch-Strasse 38**
**D-6700 Ludwigshafen(DE)**

(72) Erfinder: **Hamprecht, Gerhard, Dr.**
**Rote-Turm-Strasse 28**
**D-6940 Weinheim(DE)**

(72) Erfinder: **Varwig, Juergen, Dr.**
**Bitterstrasse 21**
**D-6900 Heidelberg(DE)**

(72) Erfinder: **Wuerzer, Bruno, Dr.**
**Ruedigerstrasse 13**
**D-6701 Otterstadt(DE)**

(72) Erfinder: **Meyer, Norbert, Dr.**
**Dossenheimer Weg 22**
**D-6802 Ladenburg(DE)**

(54) **4H-Pyrido2,3-d1,3oxazin-4-on-derivate, Verfahren zu ihrer Herstellung und ihre Verwendung zur Bekämpfung unerwünschten Pflanzenwuchses.**

(57) 4H-Pyrido[2,3-d] [1,3]oxazin-4-on-derivate der Formel

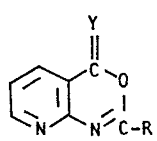

in der Y für Sauerstoff oder Schwefel und R für einen durch Halogen, Halogenalkyl, Halogenalkoxy, Halogenalkylmercapto, Halogenalkylsulfinyl oder Halogenalkylsulfonyl substituierten Phenylrest stehen,
Verfahren zu ihrer Herstallung und ihre Verwendung zur Bekämpfung unerwünschten Pflanzenwuchses.

EP 0 170 203 A2

4H-Pyrido[2,3-d][1,3]oxazin-4-on-derivate, Verfahren zu ihrer Herstellung und ihre Verwendung zur Bekämpfung unerwünschten Pflanzenwuchses

Die vorliegende Erfindung betrifft 4H-Pyrido[2,3-d][1,3]oxazin-4-on-derivate, Verfahren zu ihrer Herstellung, Herbizide, die diese Verbindungen als Wirkstoffe enthalten, ein Verfahren zur Bekämpfung unerwünschten Pflanzenwuchses mit diesen Verbindungen sowie die Verwendung dieser Verbindungen als herbizide Mittel.

Substituierte 4H-Pyrido[2,3-d][1,3]oxazin-4-one sind als pharmakologisch wirksame Verbindungen (JP-A-50 196/1978) oder hierfür geeignete Zwischenprodukte bekannt (EP-A-54 132). Weiterhin sind 4H-3,1-Benzoxazin-4-on-derivate Zwischenprodukte für die Synthese pharmakologisch wirksamer Verbindungen (DE-A-1 670 375, DE-A-2 556 590). Außerdem sind sie herbizid wirksam (BE-A-648 259, US-A-3 970 652, EP-A-17 931).

Es wurde gefunden, da 4H-Pyrido[2,3-d][1,3]oxazin-4-on-derivate der Formel Ia

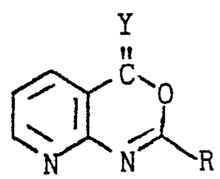

(Ia),

in der
Y für Sauerstoff oder Schwefel und
R für einen gegebenenfalls durch Halogen, $C_1$-$C_4$-Halogenalkyl, $C_1$-$C_4$-Halogenalkoxy, $C_1$-$C_4$-Halogenalkylmercapto, $C_1$-$C_4$-Halogenalkinylsulfinyl oder $C_1$-$C_4$-Halogenalkylsulfonyl o-, m- oder p-substituierten Phenylrest stehen,

neben einer guten herbiziden Wirkung eine wesentlich bessere Kulturpflanzenverträglichkeit zeigen als herbizide Mittel, die bekannte Benzoxazine als Wirkstoffe enthalten.

Kg/St

R in Formel Ia bedeutet einen Phenylrest, der gegebenenfalls folgende Substituenten tragen kann:
Fluor, Chlor, Brom, Jod, Trifluormethyl, 1,1,1-Trifluorethyl, 1,1,1,3,3,3-Hexafluorpropyl, Chlormethoxy, Fluormethoxy, Difluormethoxy, Difluorchlormethoxy, Fluordichlormethoxy, Trifluormethoxy, Trichlormethoxy, 1,1,2,2-Tetrafluorethoxy, 1,1,2-Trifluor-2-chlorethoxy, 1,1,2-Trifluor-2-chlorethoxy, 1,1,2-Trifluor-2-bromethoxy, 1,1,2,3,3,3-Hexafluorn-propyloxy, Pentafluorethoxy, Hexafluorisopropoxy, Fluordichlormethyl, Difluorchlormethyl, Difluormethylmercapto, Trifluormethylmercapto, Dichlorfluormethylmercapto, Chlordifluormethylmercapto, Pentafluorethylmercapto, 1,1,2,2-Tetrafluorethylmercapto, Chlormethylsulfonyl, Difluormethylsulfonyl, Trifluormethylsulfonyl, Pentafluorethylsulfonyl, Trifluormethylsulfinyl.

Die 4H-Pyrido[2,3-d][1,3]oxazin-4-on-derivate der Formel I

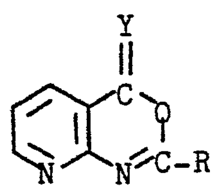

(I),

in der
Y für Sauerstoff oder Schwefel und
R für einen durch Halogen, $C_1$-$C_4$-Halogenalkyl, mit Ausnahme von Trifluormethyl, $C_1$-$C_4$-Halogenalkoxy, $C_1$-$C_4$-Halogenalkylmercapto, $C_1$-$C_4$-Halogenalkylsulfinyl oder $C_1$-$C_4$-Halogenalkylsulfonyl o-, m- oder p-substituierten Phenylrest stehen,
sind neu.

Man erhält die 4H-Pyrido[2,3-d][1,3]oxazin-4-on-derivate der Formel I dadurch, daß man eine Säure der Formel II

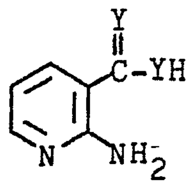

(II),

in der

Y die oben genannte Bedeutung hat, mit mindestens dem zweifachen molaren Überschuß eines Carbonsäurehalogenids der Formel III

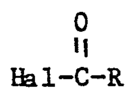

(III),

in der

R die oben genannte Bedeutung hat und Hal. für Halogen, vorzugsweise Fluor, Chlor oder Brom, insbesondere Chlor, steht, in einem aromatischen tertiären Amin als Lösungsmittel bei einer Temperatur im Bereich zwischen 10 und 90°C umsetzt.

Zweckmäßigerweise läßt man einen zweifachen Überschuß des Carbonsäurehalogenids der Formel III in eine Lösung der gegebenenfalls substituierten Aminonicotinsäure der Formel II in der 5- bis 25-fachen molaren Menge eines aromatischen Amins, bezogen auf Aminonicotinsäure, bei einer Temperatur zwischen 10 und 60°C einlaufen und rührt dann 30 bis 180 Minuten bei 25 bis 90°C nach (JP-A-50 196/1978 und J. Chem. Soc. (C) 1968, 1593). Zur Aufarbeitung kann dann Eiswasser eingerührt und vom ausgefallenen Niederschlag abgesaugt werden. Ebenso können auch das Carbonsäurehalogenid vorgelegt und die Aminonicotinsäure der Formel II zugegeben werden.

Geeignete aromatische tertiäre Amine sind beispielsweise Pyridin, α,β-,γ-Picolin, Lutidin, Chinolin und Acridin.

Die 4H-Pyrido[2,3-d][1,3]oxazin-4-on-derivate der Formel I können auch dadurch erhalten werden, daß man eine Säure der Formel II

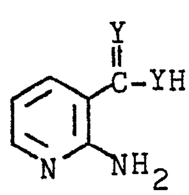

(II),

in der
Y die oben genannte Bedeutung hat, oder ein Alkalimetall- oder Erdalkalimetallsalz dieser Aminonicotinsäure mit ungefähr stöchiometrischen Mengen eines Carbonsäurehalogenids der Formel III

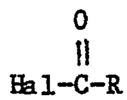 (III),

in der
R die oben genannte Bedeutung hat und Hal für Halogen steht, in einem inerten organischen Lösungsmittel oder in Wasser und gegebenenfalls in Anwesenheit eines Säureacceptors bei einer Temperatur im Bereich von 0 bis 90°C zu einem Carbonamid der Formel IV

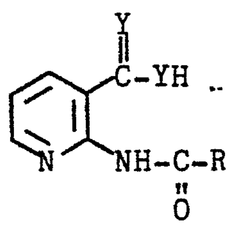 (IV),

in der
R und Y jeweils die obengenannte Bedeutung haben, umsetzt und dieses dann in Gegenwart eines wasserentziehenden MIttels bei einer Temperatur zwischen 30 und 150°C cyclisiert.

Geeignete inerte Lösungsmittel sind Kohlenwasserstoffe, wie Ligroin, Benzin, Toluol, Pentan, Hexan, Cyclohexan, Petrolether, Halogenkohlenwasserstoffe, wie Methylenchlorid, Chloroform, Tetrachlorkohlenstoff, 1,1- und 1,2-Dichlorethan, 1,1,1- und 1,1,2-Trichlorethan, Chlorbenzol, o-, m-, p-Dichlorbenzol, o-, m-, p-Chlortoluol, Nitrokohlenwasserstoffe, wie Nitrobenzol, Nitroethan, o-, m-, p-Chlornitrobenzol, Nitrile, wie Acetonitril, Butyronitril, Isobutyronitril, Ether, wie Diethylether, Di-n-propylether, Tetrahydrofuran, Dioxan, Ester wie Acetessigester, Ethylacetat oder Isobutylacetat, oder Amide, wie Formamid, N-Methylformamid oder N,N-Dimethylformamid.

Als Säureacceptoren können alle üblichen Säurebindemittel verwendet werden. Hierzu gehören vorzugsweise Alkalihydroxide, Alkalicarbonate und tertiäre organische Basen. Als besonders geeignet seien im einzelnen genannt:

Natriumhydroxid, Natriumcarbonat, Natriumhydrogencarbonat, Triethylamin, Pyridin, Trimethylamin, $\alpha$-,ß-,$\gamma$-Picolin, Lutidin, N,N-Dimethylanilin, N,N-Dimethylcyclohexylamin, Chinolin, Tri-n-propylamin und Tri-n-butylamin. Das Säurebindemittel wird zweckmäßigerweise in äquivalenten Mengen, bezogen auf Carbonsäurehalogenid der Formel III, eingesetzt.

Als wasserentziehende Mittel können symmetrische und gemischte Carbonsäureanhydride, wie Essigsäureanhydrid, Propionsäureanhydrid, Buttersäureanhydrid, Ameisensäureessigsäureanhydrid, Ameisensäurepropionsäureanhydrid oder Eissigsäurepropionsäureanhydrid, ferner Dicyclohexylcarbodiimid, ferner Phosgen, Thionylchlorid, Phosphorpentachlorid, Phosphortrichlorid, Phosphoroxychlorid oder Phosphorpentoxid verwendet werden. Die Cyclisierung wird unter Zusatz der 1- bis 10-fach-äquivalenten Menge an wasserentziehendem Mittel, bezogen auf Carbonamid der Formel IV, durchgeführt.

Die Ausgangsstoffe der Formeln II und III werden in ungefähr stöchiometrischem Verhältnis eingesetzt, d.h. in einem Unter- bzw. Überschuß von bis zu 10 % Ausgangsstoff der Formel III gegenüber Ausgangsstoff der Formel II.

Zweckmäßigerweise wird das Verfahren so durchgeführt, daß man über zwei Zuführungen das Carbonsäurehalogenid der Formel III und die äquivalente Menge an Säureacceptor bei einer Temperatur zwischen 0 und 60°C zu einer ungefähr äquivalenten Menge der Aminonicotinsäure der Formel II bzw. ihres Salzes in einem inerten organischen Lösungsmittel bzw. in Wasser zulaufen läßt. Dann rührt man 1/4 bis 14 Stunden bei 20 bis 90°C nach. Das Reaktionsgemisch wird dann gegebenenfalls eingeengt, in der Wärme mit 5N Salzsäure angesäuert, abgekühlt und abgesaugt (J. Org. Chem. 9, 396, (1944)), wobei eine N-Acyl-2-amino-nicotinsäure erhalten wird. Diese kann man in Gegenwart der 5- bis 10-fachen Menge Acetanhydrid durch Rühren unter Rückfluß - gegebenenfalls unter Abdestillation der entstandenen

Essigsäure - zu dem gewünschten 4H-Pyrido[2,3-d][1,3]oxazin-4-on-derivat cyclisieren. Zur Aufarbeitung entfernt man hierbei überschüssiges Acetanhydrid am Rotationsverdampfer unter vermindertem Druck und kristallisiert gegebenenfalls zur Reinigung um. Anstelle der Aminonicotinsäure kann auch das Carbonsäurehalogenid vorgelegt werden.

Anstelle von Acetanhydrid kann man jedoch auch mit der 1- bis 4-fachen äquivalenten Menge Dicyclohexylcarbodiimid, ferner Phosgen, Thionylchlorid, Phosphorpentachlorid, Phosphortrichlorid, Phosphoroxychlorid oder Phosphorpentoxid, bei 30 bis 150°C cyclisieren.

Zur Isolierung der 4H-Pyrido[2,3-d][1,3]oxazin-4-on-derivate der Formel I aus der Reaktionsmischung kann man diese mit Wasser, verdünntem Alkali oder verdünnter Säure zur Abtrennung von Nebenprodukten, wie nicht umgesetzter Aminonicotinsäure, Säurechlorid bzw. Basenhydrochlorid, behandeln, trocknen und dann einengen. Gegebenenfalls können die Endprodukte auch durch Umkristallisation oder Chromatographie gereinigt werden.

Beispiel 1

2-(3'-Trifluormethylthio-phenyl)-4H-pyrido[2,3-d][1,3]oxazin-4-on

33 Teile 3-Trifluormethylthio-benzoylfluorid und 15 Teile Triethylamin wurden gleichzeitig under Rühren bei 25 bis 30°C innerhalb 10 Minuten zu einer Suspension von 20 Teilen 2-Aminonicotinsäure in 180 Teilen Methylenchlorid gegeben. Nach dem Rühren über Nacht bei 25°C wurde noch eine Stunde bei 41°C gerührt. Der Kolbeninhalt wurde mit Wasser und zweimal mit 1N Salzsäure extrahiert und dann eingeeingt, wobei 46 Teile 2-N-(3'-Trifluormethylthio-benzoyl)-aminonicotinsäure vom Fp. 138 - 150°C erhalten wurden.

Hiervon wurden 43 Teile in 220 Teilen 1,2-Dichlorethan vorgelegt und mit 26 Teilen Thionylchlorid versetzt. Das Reaktionsgemisch wurde anschließend 6 Stunden unter Rückfluß gerührt und nach dem Abkühlen in Eiswasser eingerührt. Die organische Phase wurde abgetrennt, mit 10 %iger Natriumcarbonatlösung extrahiert, getrocknet und über neutrales Aluminiumoxid

chromatographiert. Nach dem Einengen wurden 25 Teile 2-(3'-Trifluormethylthio-phenyl)-4H-pyrido[2,3-d][1,3]oxazin-4-on vom Fp. 141 - 143°C erhalten.

Beispiel 2

2-(4'-Chlordifluormethoxy-phenyl)-4H-pyrido[2,3-d][1,3]oxazin-4-on

33 Teile 4-Chlordifluormethoxy-benzoylfluorid und 13,5 Teile -Picolin wurden bei 20 bis 35°C unter Rühren innerhalb 10 Minuten zu einer Suspension von 20 Teilen 2-Aminonicotinsäure in 250 Teilen 1,2-Dichlorethan gegeben. Das Reaktionsgemisch wurde jeweils 4 Stunden lang bei 25 und 83°C gerührt und dann unter vermindertem Druck eingeeingt. Der Rückstand wurde mit 0,5N Salzsäure verrührt, abgesaugt, mit Wasser und mit 30 Teilen Methyl-tert-butylether gewaschen, wobei 36,9 Teile 2-N-(4'-Chlordifluormethoxy-benzoyl)-aminonicotinsäure vom Fp. 234 - 238°C erhalten wurden.

Hiervon werden 15 Teile in 220 Teilen 1,2-Dichlorethan vorgelegt und portionsweise mit 7 Teilen Thionylchlorid unter Rühren bei 25°C vereinigt. Das Reaktionsgemisch wird 4 Stunden bewi 81°C abgekühlt und in 300 Teile Eiswasser eingerührt. Die abgetrennte organische Phase wird einmal mit 0,5n Natronlauge extrahiert, getrocknet und über Aluminiumoxid chromatographiert. Nach dem Einengen werden 12 Teile 2-(4'-Chlordifluormethoxy-phenyl)-4H-pyrido[2,3-d][1,3]oxazin-4-on vom Fp. 111 - 115°C erhalten.

Nach entsprechenden Verfahren können folgende Pyridooxazinderivate der Formel I hergestellt werden.

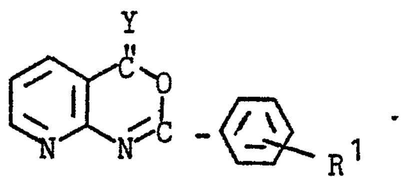

| Verbindung Nr. | $R^1$ | Y | Fp [°C] |
|---|---|---|---|
| 3 | 3-Cl | O | |
| 4 | 4-Cl | O | |
| 5 | 3-F | O | 134 - 135 |
| 6 | 4-F | O | 198 - 200 |
| 7 | 3-F | S | |
| 8 | 2-F | O | |
| 9 | 3-Br | O | |
| 10 | 4-Br | O | |
| 11 | 4-J | O | |
| 12 | 3-J | O | |
| 13 | 3-$CF_3$ | O | 179 - 182 |
| 14 | 4-$CF_3$ | O | 164 - 166 |
| 15 | 4-$CF_3$ | S | |
| 16 | 3-$CH_2$-$CF_3$ | O | |
| 17 | 3-$CH(CF_3)_2$ | O | |
| 18 | 4-$CH(CF_3)_2$ | O | |
| 19 | 3-O-$CH_2Cl$ | O | |
| 20 | 4-O-$CH_2Cl$ | O | |
| 21 | 3-O-$CHF_2$ | O | |
| 22 | 3-O-$CHF_2$ | O | 123 - 125 |
| 23 | 4-O-$CHF_2$ | O | 159 - 161 |
| 24 | 2-O-$CHF_2$ | O | |
| 25 | 3-O-$CHF_2$ | S | |
| 26 | 3-O-$CF_2Cl$ | O | 104 - 107 |
| 27 | 3-O-$CF_2Cl$ | S | |

| Verbindung Nr. | $R^1$ | Y | Fp [°C] |
|---|---|---|---|
| 28 | 2-O-$CF_2Cl$ | O | |
| 29 | 4-O-$CF_2Cl$ | S | |
| 30 | 3-O-$CFCl_2$ | O | |
| 31 | 3-O-$CF_3$ | O | 142 - 144 |
| 32 | 4-O-$CF_3$ | O | 130 - 132 |
| 33 | 3-O-$CF_3$ | S | |
| 34 | 4-O-$CCl_3$ | O | |
| 35 | 3-O-$CF_2$-$CF_2H$ | O | 147 - 148 |
| 36 | 4-O-$CF_2$-$CF_2H$ | O | 139 - 140 |
| 37 | 4-O-$CF_2$-$CF_2H$ | S | |
| 38 | 3-O-$CF_2$-CHFCl | O | 139 - 141 |
| 39 | 4-O-$CF_2$-CHFCl | O | |
| 40 | 3-O-$CF_2$-CHFBr | O | |
| 41 | 3-O-$CF_2$-CHF-$CF_3$ | O | |
| 42 | 4-O-$CF_2$-CHF-$CF_3$ | O | |
| 43 | 3-O-$CF_2$-$CF_3$ | O | |
| 44 | 3-O-$CH(CF_3)_2$ | O | |
| 45 | 3-$CF_2Cl$ | O | |
| 46 | 4-$CF_2Cl$ | O | |
| 47 | 3-$CFCl_2$ | O | |
| 48 | 4-$CFCl_2$ | O | |
| 49 | 3-S-$CHF_2$ | O | |
| 50 | 4-S-$CHF_2$ | O | |
| 51 | 3-S-$CHF_2$ | S | |
| 52 | 4-S-$CF_3$ | O | 177 - 179 |
| 53 | 3-S-$CF_3$ | S | |
| 54 | 3-S-$CF_2Cl$ | O | |
| 55 | 4-S-$CF_2Cl$ | O | |
| 56 | 3-S-$CFCl_2$ | O | |
| 57 | 4-S-$CFCl_2$ | O | |
| 58 | 3-S-$CF_2$-$CF_3$ | O | |
| 59 | 3-S-$CF_2$-$CF_2H$ | O | |
| 60 | 4-S-$CF_2$-$CF_2H$ | O | |

| Verbindung Nr. | $R^1$ | Y | Fp [°C] |
|---|---|---|---|
| 61 | $3-SO_2CH_2Cl$ | O | |
| 62 | $4-SO_2CH_2Cl$ | O | |
| 63 | $3-SO_2CHF_2$ | O | |
| 64 | $4-SO_2CHF_2$ | O | |
| 65 | $3-SO_2CF_3$ | O | |
| 66 | $3-SO_2CF_3$ | S | |
| 67 | $4-SO_2CF_3$ | O | |
| 68 | $3-SO_2CF_2CF_3$ | O | |
| 69 | $4-SO_2CF_2CF_3$ | O | |
| 70 | H | O | 142 - 144 |
| 71 | $3-SOCF_3$ | O | |
| 72 | $4-SOCF_3$ | O | |

Die 4H-Pyrido[2,3-d][1,3]oxazin-4-on-derivate der Formel Ia können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittleren Naphthalinen oder Methanol, Ethanol, Propanol, Butanol, Chloroform, Tetrachlorkohlenstoff, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldisper-

sionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonat, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Alkylarylpolyetheralkohole, Isotgridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäure, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gew.-%, vorzugsweise zwischen 0,5 und 90 Gew.-%, Wirkstoff.

Die Applikation kann im Vorauflauf- oder im Nachauflaufverfahren erfolgen. Sind die Wirkstoffe bei Nachauflaufanwendung für gewisse Kulturpflanzen weniger verträglich, so können auch Aufbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,05 bis 5 kg/ha, vorzugsweise 0,25 bis 4 kg/ha.

Die Wirkung der 4H-Pyrido[2,3-d][1,3]oxazin-4-on-derivate der Formel Ia auf das Pflanzenwachstum läßt sich durch Gewächshausversuche zeigen:

Als Kulturgefäße dienen Plastikblumentöpfe mit 300 $cm^3$ Inhalt und lehmigem Sand mit etwa 3,0 % Humus als Substrat.

Zur Nachauflaufbehandlung werden entweder direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Man zieht die Testpflanzen je nach Wuchsform erst bis zu einer Wuchshöhe von 3 bis 15 cm an und behandelt sie danach mit den aufbereiteten Wirkstoffen, die in Wasser als Verteilungsmittel suspendiert oder emulgiert und mittels fein verteilender Düsen gespritzt werden. Abhängig vom Wirkstoff können die Aufwandmengen für die Nachauflaufbehandlung variieren. Sie betragen beispielsweise 3,0, 1,0, 0,5 und 0,125 kg Wirkstoff/ha.

Die Versuchsgefäße werden im Gewächshaus aufgestellt, wobei für wärmeliebende Arten wärmere Bereiche (20 bis 35°C) und für solche gemäßigter Klimate 10 bis 20°C bevorzugt werden. Die Versuchsperiode erstreckt sich über zwei bis vier Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 100 völlige Zerstörung zumindest der oberirdischen Teile der Pflanzen.

In den Versuchen werden folgende Pflanzenartzen verwendet:

| Lateinischer Name | Deutscher Name |
|---|---|
| Avena sativa | Hafer |
| Beta vulgaris | Zuckerrübe |
| Centaurea cyanus | Kornblume |
| Chrysanthemum spp. | Wucherblume |
| Chrysanthemum coronarium | Goldblume |
| Euphorbia heterophylla | Wolfsmilcharten |
| Galium aparine | Klettenlabkraut |
| Lamium amplexicaule | Stengelumfassende Taubnessel |
| Mercurialis annua | Einjähriges Bingelkraut |
| Polygonum aviculare | Vogelknöterich |
| Solanum nigrum | Schwarzer Nachtschatten |
| Triticum aestivum | Weizen |
| Veronica spp. | Ehrenpreisarten |
| Zea mays | Mais |

Bei Nachauflaufanwendung mit einer Aufwandmenge von 3,0 kg Wirkstoff/ha zeigen beispielsweise die Verbindungen 14, 32 und 36 gegen breitblättrige Beispielpflanzen beachtliche herbizide Wirkung, während Hafer als Beispielkultur völlig ungeschädigt bleibt. Entsprechende Vergleichsmittel auf Benzoxazinonbasis, bekannt aus US-A-3 914 121 und EP-A-17 931, zeigen kaum herbizide Aktivität.

In den Beispielkulturen Zuckerrübe und Mais lassen sich unerwünschte breitblättrige Pflanzen mit einer Aufwandmenge von 1,0 kg Wirkstoff/ha der beispielhaft ausgewählten Verbindungen 14 und 15 gut bekämpfen ohne die Kulturpflanzen nennenswert zu beeinflussen. Ein Vergleichsmittel, bekannt aus US-A-3 914 121, schädigt einerseits die Zuckerrüben massiv und zeigt ein deutlich niedrigeres herbizides Wirkniveau.

Beispielsweise eignet sich die Verbindung 32, angewendet in einer Aufwandmenge von 0,5 kg Wirkstoff/ha, zur Bekämpfung eines breiten Schadpflanzenspektrums in Weizen und Mais. Für beide Kulturpflanzen ist der Wirkstoff gut verträglich.

Die beispielhaft ausgewählte Verbindung Nr. 2 bekämpft mit einer Aufwandmenge von 0,125 kg Wirkstoff/ha im Nachauflaufverfahren unerwünschte breitblättrige Unkräuter. Rüben erfahren dadurch nur eine noch akzeptable Schädigung. Ein aus der EP-A-17 931 bekanntes Vergleichsmittel dagegen wirkt auf Rüben herbizid.

In Anbetracht der guten Verträglichkeit der Wirkstoffe und der Vielseitigkeit der Applikationsmethoden können die Wirkstoffe der Formel Ia oder diese enthaltende Mittel außer bei den in den Gewächshausversuchen getesteten Nutzpflanzen noch in einer weiteren großen Zahl von Kulturpflanzen zur Beseitigung unerwünschten Pflanzenwuchses eingesetzt werden.

In Betracht kommen beispielsweise die folgenden Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |

| Botanischer Name | Deutscher Name |
|---|---|
| Carthamus tinctorius | Saflor - Färberdistel |
| Caryg illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |

| Botanischer Name | Deutscher Name |
|---|---|
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sasamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |

| Botanischer Name | Deutscher Name |
|---|---|
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die 4H-Pyrido[2,3-d][1,3]oxazin-4-on-derivate der Formel Ia und ihre Salze mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere herbizide Wirkstoffe in Betracht.

Eine Reihe von Wirkstoffen, die zusammen mit den neuen Verbindungen für verschiedene Gebiete sinnvolle Mischungen ergeben, werden beispielhaft aufgeführt:

3-(1-Methylethyl)-1H-2,1,3-benzothiadiazin-4(3H)-on-2,2-dioxid und Salze
3-(1-Methylethyl)-8-chlor-1H-2,1,3-benzothiadiazin-4(3H)-on-2,2-dioxid und Salze
3-(1-Methylethyl)-8-fluor-1H-2,1,3-benzothiadiazin-4(3H)-on-2,2-dioxid und Salze
3-(1-Methylethyl)-8-methyl-1H-2,1,3-benzothiadiazin-4(3H)-on-2,2-dioxid und Salze

1-Methoxymethyl-3-(1-methylethyl)-2,1,3-benzothiadiazin-4(3H)-on-2,2-dioxid
1-Methoxymethyl-8-chlor-3-(1-methylethyl)-2,1,3-benzothiadiazin-4(3H)-on-2,2-dioxid

1-Methoxymethyl-8-fluor-3-(1-methylethyl)-2,1,3-benzothiadiazin-4(3H)-on-2,2-dioxid

1-Cyan-8-chlor-3-(1-methylethyl)-2,1,3-benzothiadiazin-4(3H)-on-2,2-dioxid

1-Cyan-8-fluor-3-(1-methylethyl)-2,1,3-benzothiadiazin-4(3H)-on-2,2-dioxid

1-Cyan-8-methyl-3-(1-methylethyl)-2,1,3-benzothiadiazin-4(3H)-on-2,2-dioxid

1-Azidomethyl-3-(1-methylethyl)-2,1,3-benzothiadiazin-4(3H)-on-2,2-dioxid

3-(1-Methylethyl)-1H-(pyridino-[3,2-e]2,1,3-thiadiazin-(4)-on-2,2-dioxid

1-Cyan-3-(1-methylethyl)-2,1,3-benzothiadiazin-4(3H)-on-2,2-dioxid

N-(1-Ethylpropyl)-2,6-dinitro-3,4-dimethylanilin

N-(1-Methylethyl)-N-ethyl-2,6-dinitro-4-trifluormethyl-anilin

N-n-Propyl-N-ß-chlorethyl-2,6-dinitro-4-trifluormethyl-anilin

N-n-Propyl-N-cyclopropylmethyl-2,6-dinitro-4-trifluor-methyl-anilin

N,N-Di-n-propyl-2,6-dinitro-3-amino-4-trifluormethylanilin

N,N-Di-n-propyl-2,6-dinitro-4-methyl-anilin

N,N-Di-n-propyl-2,6-dinitro-4-methylsulfonyl-anilin

N,N-Di-n-propyl-2,6-dinitro-4-aminosulfonyl-anilin

N,N-Di-beta-chlorethyl-2,6-dinitro-4-methyl-anilin

N-Ethyl-N-(2-methylallyl)-2,6-dinitro-4-trifluormethyl-anilin

N-Methylcarbaminsäure-3,4-dichlorbenzylester

N-Methylcarbaminsäure-2,6-di-tert.butyl-4-methylphenyl-ester

N-Phenylcarbaminsäure-isopropylester

N-3-Chlorphenylcarbaminsäure-isopropylester

N-3-Chlorphenylcarbaminsäure-butin-1-yl-3-ester

N-3-Chlorphenylcarbaminsäure-4-chlor-butin-2-yl-ester

N-3,4-Dichlorphenylcarbaminsäure-methylester

N-(4-Amino-benzolsulfonyl)-carbaminsäure-methylester

O-(N-Phenylcarbamoyl)-propanonoxim

N-Ethyl-2-(phenylcarbamoyl)-oxypropionsäureamid

3'-N-Isopropyl-carbamoyloxy-propionanilid

Ethyl-N-[3-(N'-phenylcarbamoyloxy)-phenyl]-carbamat
Methyl-N-[3-(N'-methyl-N'-phenylcarbamoyloxy)-phenyl]-carbamat
Isopropyl-N-[3-(N'-ethyl-N'-phenylcarbamoyloxy)-phenyl]-carbamat
Methyl-N-[3-(N'-3-methylphenylcarbamoyloxy)-phenyl]-carbamat
Ethyl-N-[3-(N'-3-chlor-4-fluorphenylcarbamoyloxy)-phenyl]-carbamat
Ethyl-N-[3-(N'-3,4-difluorphenylcarbamoyloxy)-phenyl]-carbamat
N,N-Diethyl-thiolcarbaminsäure-p-chlorbenzylester
N,N-Di-n-propyl-thiolcarbaminsäure-ethylester
N,N-Di-n-propyl-thiolcarbaminsäure-n-propylester
N,N-Di-isopropyl-thiolcarbaminsäure-2,3-dichlorallylester
N,N-Di-isopropyl-thiolcarbaminsäure-2,3,3-trichlorallylester
N,N-Di-isopropyl-thiolcarbaminsäure-3-methyl-5-isoxazolyl-methylester
N,N-Di-isopropyl-thiolcarbaminsäure-3-ethyl-5-isoxazolyl-methylester
N,N-Di-sec.butyl-thiolcarbaminsäure-ethylester
N,N-Di-sec.butyl-thiolcarbaminsäure-benzylester
N-Ethyl-N-cyclohexyl-thiolcarbaminsäure-ethylester
N-Ethyl-N-bicyclo[2.2.1]heptyl-thiolcarbaminsäureethylester
S-Ethyl-hexahydro-1H-azepin-1-carbothiolat
S-Ethyl-3-methylhexahydro-1-H-azepin-1-carbothiolat

N-Ethyl-N-n-butyl-thiolcarbaminsäure-n-propylester
N,N-Dimethyl-dithiocarbaminsäure-2-chlorallylester
N-Methyl-dithiocarbaminsäure-Natriumsalz
Trichloressigsäure-Natriumsalz
Alpha,alpha-Dichlorpropionsäure-Natriumsalz
Alpha,alpha-Dichlorbuttersäure-Natriumsalz
Alpha,alpha,beta,beta-Tetrafluorpropionsäure-Natriumsalz
Alpha-Methyl-alpha,beta-dichlorpropionsäure-Natriumsalz
Alpha-Chlor-beta-(4-chlorphenyl)-propionsäure-methylester
Alpha,beta-Dichlor-beta-phenylpropionsäure-methylester
Benzamido-oxy-essigsäure
2,3,5-Trijodbenzoesäure (Salze, Ester, Amide)
2,3,6-Trichlorbenzoesäure (Salze, Ester, Amide)
2,3,5,6-Tetrachlorbenzoesäure (Salze, Ester, Amide)
2-Methoxy-3,6-dichlorbenzoesäure (Salze, Ester, Amide)
2-Methoxy-3,5,6-trichlorbenzoesäure (Salze, Ester, Amide)
3-Amino-2,5,6-trichlorbenzoesäure (Salze, Ester, Amide)

O,S-Dimethyl-tetrachlor-thioterephthalat
Dimethyl-2,3,5,6-tetrachlor-terephthalat
Dinatrium-3,6-endoxohexahydro-phthalat
4-Amino-3,5,6-trichlor-picolinsäure (Salze)
2-Cyan-3-(N-methyl-N-phenyl)-amino-acrylsäureethylester
2-[4-(4'-Chlorphenoxy)-phenoxy]-propionsäureisobutylester
2-[4-(2',4'-Dichlorphenoxy)-phenoxy]-propionsäuremethylester
2-[4-(4'-Trifluormethylphenoxy)-phenoxy]-propionsäure-methylester
2-[4-(2'-Chlor-4'-trifluorphenoxy)-phenoxy]-propionsäureNatriumsalz
2-[4-(3',5'-Dichlorpyridyl-2-oxy)-phenoxy]-propionsäureNatriumsalz

2-(N-Benzoyl-N-(3,4-dichlorphenyl)-amino)-propionsäureethylester
2-(N-Benzoyl-3-chlor-4-fluorphenylamino)-propionsäure-methylester
2-(N-Benzoyl-3-chlor-4-fluorphenylamino)-propionsäureisopropylester

2-Chlor-4-ethylamino-6-isopropylamino-1,3,5-triazin
2-Chlor-4-ethylamino-6-(amino-2'-propionitril)-1,3,5-triazin
2-Chlor-4,6-bisethylamino-1,3,5-triazin
2-Chlor-4,6-bisisopropylamino-1,3,5-triazin
2-Chlor-4-isopropylamino-6-cyclopropylamino-1,3,5-triazin

2-Azido-4-methylamino-6-isopropylamino-1,3,5-triazin
2-Methylthio-4-ethylamino-6-isopropylamino-1,3,5-triazin
2-Methylthio-4-ethylamino-6-tert.butylamino-1,3,5-triazin
2-Methylthio-4,6-bisethylamino-1,3,5-triazin
2-Methylthio-4,6-bisisopropylamino-1,3,5-triazin
2-Methoxy-4-ethylamino-6-isopropylamino-1,3,5-triazin
2-Methoxy-4,6-bisethylamino-1,3,5-triazin
2-Methoxy-4,6-bisisopropylamino-1,3,5-triazin
4-Amino-6-tert.butyl-3-methylthio-4,5-dihydro-1,2,4-triazin-5-on
4-Amino-6-phenyl-3-methyl-4,5-dihydro-1,2,4-triazin-5-on
4-Isobutylidenamino-6-tert.butyl-3-methylthio-4,5-dihydro-1,2,4-triazin-5-on
1-Methyl-3-cyclohexyl-6-dimethylamino-1,3,5-triazin-2,4-dion

3-tert.Butyl-5-chlor-6-methyluracil
3-Isopropyl-5-brom-6-methyluracil

3-sec.Butyl-5-brom-6-methyluracil
3-Cyclohexyl-5,6-trimethylenuracil

2-Methyl-4-(3'-trifluormethylphenyl)-tetrahydro-1,2,4-oxadiazin-3,5-dion
2-Methyl-4-(4'-fluorphenyl)-tetrahydro-1,2,4-oxadiazin-3,5-dion
3-Amino-1,2,4-triazol

1-(4-Chlorphenoxy-3,3-dimethyl-1-(H-1,2,4-triazolyl)-2-butanon
N,N-Diallylchloracetamid
N-Isopropyl-2-chloracetanilid
N-(1-Methyl-propin-2-yl)-2-chloracetanilid

2-Methyl-6-ethyl-N-ethoxymethyl-2-chloracetanilid
2-Methyl-6-ethyl-N-(2-methoxy-1-methylethyl)-2-chloracetanilid
2-Methyl-6-ethyl-N-(isopropoxycarbonylethyl)-2-chloracet-anilid
2-Methyl-6-ethyl-N-(pyrazolyl-methyl)-2-chloracetanilid
2,6-Dimethyl-N-(pyrazolyl-methyl)-2-chloracetanilid
2,6-Dimethyl-N-(4-methylpyrazolyl-methyl)-2-chloracetanilid
2,6-Dimethyl-N-(1,2,4-triazolyl-methyl)-2-chloracetanilid
2,6-Dimethyl-N-(3,5-dimethylpyrazolyl-methyl)-2-chloracetanilid
2,6-Dimethyl-N-(1,3-dioxolan-2-yl-methyl)-2-chloracetanilid
2,6-Dimethyl-N-(2-methoxyethyl)-2-chloracetanilid
2,6-Dimethyl-N-isobutoxymethyl-2-chloracetanilid
2,6-Diethyl-N-methoxymethyl-2-chloracetanilid
2,6-Diethyl-N-(n-butoxymethyl)-2-chloracetanilid
2,6-Diethyl-N-ethoxycarbonylmethyl-2-chloracetanilid
2,3-Dimethyl-N-isopropyl-2-chloracetanilid
2,6-Diethyl-N-(2-n-propoxy-ethyl)-2-chloracetanilid

alpha-(2-Methyl-4-chlorphenoxy)-N-methoxy-acetamid
2-(alpha-Naphthoxy)-N,N-diethylpropionamid
2,2-Diphenyl-N,N-dimethylacetamid
alpha-(3,4,5-Tribrompyrazolyl)-N,N-dimethylpropionamid
N-(1,1-Dimethylprop-2-inyl)-3,5-dichlorbenzamid
N-Naphth-1-yl-phthalamidsäure

Propionsäure-3,4-dichloranilid
Cyclopropancarbonsäure-3,4-dichloranilid
Methacrylsäure-3,4-dichloranilid
2-Methylpentancarbonsäure-3,4-dichloranilid
5-Acetamido-2,4-dimethyltrifluormethan-sulfonanilid
5-Acetamido-4-methyl-trifluormethan-sulfonanilid

2-Propionyl-amino-4-methyl-5-chlor-thiazol
O-(Methylaminosulfonyl)-glykolsäure-hexamethylenimid
2,6-Dichlor-thiobenzamid
2,6-Dichlorbenzonitril

3,5-Dibrom-4-hydroxy-benzonitril (Salze)
3,5-Diiod-4-hydroxy-benzonitril (Salze)
3,5-Dibrom-4-hydroxy-O-2,4-dinitrophenylbenzaldoxim (Salze)
Pentachlorphenyl-Natriumsalz
2,4-Dichlorphenyl-4'-nitrophenylether
2,4,6-Trichlorphenyl-4'-nitrophenylether
2-Fluor-4,6-dichlorphenyl-4'-nitrophenylether
2-Chlor-4-trifluormethylphenyl-4'-nitrophenylether
2,4'-Dinitro-4-trifluormethyl-diphenylether
2,4-Dichlorphenyl-3'-methoxy-4'-nitro-phenylether
2-Chlor-4-trifluormethylphenyl-3'-ethoxy-4'-nitro-phenylether
2-Chlor-4-trifluormethylphenyl-3'-carboxy-4'-nitro-phenylether (Salze)
2,4-Dichlorphenyl-3'-methoxycarbonyl-4'-nitro-phenylether
2-(3,4-Dichlorphenyl)-4-methyl-1,2,4-oxadiazolidin-3,5-dion
2-(3-Isopropylcarbamoyl-oxyphenyl)-4-methyl-1,2,4-oxadiazolidin-3,5-dion
2-Phenyl-3,1-benzoxazinon-(4)
3-(4-Bromphenyl)-3,4,5,9,10-pentaazatetracyclo-[5,4,1,$0^{2,6}$,$0^{8,11}$]-dodeca-3,9-dien
2-Ethoxy-2,3-dihydro-3,3-dimethyl-5-benzofuranyl-methan-sulfonat
2-Methyl-4,6-dinitrophenol (Salze, Ester)
3-(4-Chlorphenyl)-3,4,5,9,10-pentaazatetracyclo-[5,4,1,$0^{2,6}$,$0^{8,1}$]-dodeca-3,9-dien
2-sec.Butyl-4,6-dinitrophenol (Salze, Ester)
2-sec.Butyl-4,6-dinitrophenol-acetat

2-tert.Butyl-4,6-dinitrophenol-acetat
2-tert.Butyl-4,6-dinitrophenol (Salze)
2-tert.Butyl-5-methyl-4,6-dinitrophenol (Salze)
2-tert.Butyl-5-methyl-4,6-dinitrophenol-acetat
2-sec.Amyl-4,6-dinitrophenol (Salze, Ester)
1-(alpha,alpha-Dimethylbenzyl)-3-(4-methylphenyl)-harnstoff
1-Phenyl-3-(2-methylcyclohexyl)-harnstoff
1-(4-Chlorphenyl)-3,3-dimethyl-harnstoff
1-(4-Chlorphenyl)-3-methyl-3-(butin-1-yl-3)-harnstoff
1-(3,4-Dichlorphenyl)-3,3-dimethyl-harnstoff
1-(3,4-Dichlorphenyl)-3-methyl-3-n-butyl-harnstoff
1-(4-i-Propylphenyl)-3,3-dimethyl-harnstoff
1-(3-Trifluormethylphenyl)-3,3-dimethyl-harnstoff
1-(alpha,alpha,beta,beta-Tetrafluorethoxyphenyl)-3,3-dimethyl-harnstoff
1-(3-tert.Butylcarbamoyloxy-phenyl)-3,3-dimethyl-harnstoff
1-(3-Chlor-4-methylphenyl)-3,3-dimethyl-harnstoff
1-(3-Chlor-4-methoxyphenyl)-3,3-dimethyl-harnstoff
1-[4(4'-Chlorphenoxy)-phenyl]-3,3-dimethyl-harnstoff
1-[4(4'-Methoxyphenoxy)-phenyl]-3,3-dimethyl-harnstoff
1-Cyclooctyl-3,3-dimethyl-harnstoff
1-(Hexahydro-4,7-methanindan-5-yl)-3,3-dimethyl-harnstoff
1-[1- oder 2-(3a,4,5,7,7a-Hexahydro)-4,7-methanoindanyl]-3,3-dimethyl--harnstoff
1-(4-Chlorphenyl)-3-methyl-3-methoxy-harnstoff
1-(4-Bromphenyl)-3-methyl-3-methoxy-harnstoff
1-(3,4-Dichlorphenyl)-3-methyl-3-methoxy-harnstoff
1-(3-Chlor-4-bromphenyl)-3-methyl-3-methoxy-harnstoff
1-(2-Benzthiazolyl)-1,3-dimethyl-harnstoff
1-(2-Benzthiazolyl)-3-methyl-harnstoff
1-(5-Trifluormethyl-1,3,4-thiadiazolyl)-1,3-dimethyl-harnstoff
Imidazolidin-2-on-1-carbonsäure-isobutylamid
1,2-Dimethyl-3,5-diphenylpyrazolium-methylsulfat
1,3-Dimethyl-4-(3,4-dichlorbenzoyl)-5-(4-methylphenylsulfonyloxy)-pyrazol
2,3,5-Trichlor-pyridinol-(4)

1-Methyl-3-phenyl-5-(3'-trifluormethylphenyl)-pyridon-(4)
1-Methyl-4-phenyl-pyridiniumchlorid
1,1-Dimethylpyridiniumchlorid
1,1'-Dimethyl-4,4'-dipyridylium-di(methylsulfat)
1,1'-Di(3,5-dimethylmorpholin-carbonylmethyl)-4,4'-dipyridylium-dichlorid
1,1'-Ethylen-2,2'-dipyridylium-dibromid
3-[1-(N-Ethoxyamino)-propyliden]-6-ethyl-3,4-dihydro-2-H-pyran-2,4-dion
3-[1-(N-Allyloxyamino)-propyliden]-6-ethyl-3,4-dihydro-2-H-pyran-2,4-dion
2-[1-(N-Allyloxyamino)-propyliden]-5,5-dimethylcyclohexan-1,3-dion (Salze)
2-[1-(N-Allyloxyamino-butyliden]-5,5-dimethylcyclohexan-1,3-dion (Salze)
2-[1-(N-Allyloxyamino-butyliden]-5,5-dimethyl-4-methoxycarbonyl-cyclohexan-1,3-dion (Salze)
2,4-Dichlorphenoxyessigsäure (Salze, Ester, Amide)
2-Methyl-4-chlorphenoxyessigsäure (Salze, Ester, Amide)
3,5,6-Trichlor-2-pyridinyl-oxyessigsäure (Salze, Ester, Amide)

Alpha-Naphthoxyessigsäuremethylester
2-(2,4-Dichlorphenoxy)-propionsäure (Salze, Ester, Amide)
2-(2-Methyl-4-chlorphenoxy)-propionsäure (Salze, Ester, Amide)
4-(2,4-Dichlorphenoxy)-buttersäure (Salze, Ester, Amide)
4-(2-Methyl-4-chlorphenoxy)-buttersäure (Salze, Ester, Amide)
9-Hydroxyfluoren-carbonsäure-(9) (Salze, Ester)
2,3,6-Trichlorphenyl-essigsäure (Salze, Ester)
4-Chlor-2-oxo-benzothiazolin-3-yl-essigsäure (Salze, Ester)
Gibellerinsäure (Salze)
Dinatrium-methylarsonat
Mononatriumsalz der Methylarsonsäure

N-Phosphon-methyl-glycin (Salze)
N,N-Bis(phosphonmethyl)-glycin (Salze)
2-Chlorethanphosphonsäure-2-chlorethylester
Ammonium-ethyl-carbamoyl-phosphonat
0,0-Di-n-butyl-(1-n-butylamino-cyclohexyl)-phosphonat
Trithiobutylphosphit

O,O-Diisopropyl-5-(2-benzolsulfonylamino-ethyl)-phosphordithioat
5-tert.Butyl-3-(2,4-dichlor-5-isopropoxyphenyl)-1,3,4-oxadiazolon-(2)
4,5-Dichlor-2-trifluormethyl-benzimidazol (Salze)
1,2,3,6-Tetrahydropyridazin-3,6-dion (Salze)
Bernsteinsäure-mono-N,N-dimethylhydrazid (Salze)
(2-Chlorethyl)-trimethyl-ammoniumchlorid
(2-Methyl-4-phenylsulfonyl)-trifluormethansulfonanilid
Ammoniumrhodanid
Calciumcyanamid

2-Chlor-4-trifluormethylphenyl-3'-ethoxycarbonyl-4'-nitrophenylether
1-(4-Benzyloxyphenyl)-3-methyl-3-methoxyharnstoff
2-[1-(2,5-Dimethylphenyl)-ethylsulfonyl]-pyridin-N-oxid
N-Benzyl-N-isopropyl-trimethylacetamid
2-[4-(4'-Chlorphenoxymethyl)-phenoxy]-propionsäuremethylester
2-[4-(5'-Brompyridyl-2-oxy)-phenoxy]-propionsäureethylester
2-[4-(5'-Iodpyridyl-2-oxy)-phenoxy]-propionsäure-n-butylester
2-Chlor-4-trifluormethylphenyl-3'-(2-fluorethoxy)-4'-nitro-phenylether
2-Chlor-4-trifluormethylphenyl-3'-ethoxycarbonylmethylthio-4'-nitrophenylether.

2,4,6-Trichlorphenyl-3'-ethoxycarbonyl)methylthio-4'-nitrophenylether
2-[1-(N-Ethoxyamino)-butyliden]-5-(2-ethylthiopropyl)-3-hydroxy-cyclohexen-(2)-on-(1) (Salze)
2-[1-(N-Ethoxyamino)-butyliden]-5-(2-phenylthiopropyl)-3-hydroxy-cyclohexen-(2)-on-(1) (Salze)

4-[4-(4'-Trifluormethyl)-phenoxy]-penten-2-carbonsäureethylester
2-Chlor-4-trifluormethyl-3'-methoxycarbonyl-4'-nitrophenylether
2,4-Dichlorphenyl-3'-carboxy-4'-nitrophenylether (Salze)
4,5-Dimethoxy-2-(3-alpha,alpha,beta-trifluor-beta-bromethoxyphenyl)-3-(2H)-pyridazinon
2,4-Dichlor-3'[2-(2-ethoxy-ethoxy)-ethoxy]-4'-nitro-diphenyl-ether
2,3-Dihydro-3,3-dimethyl-5-benzofuranyl-ethansulfonat
N-[4-Methoxy-6-methyl-1,3,5-triazin-2-yl-aminocarbonyl]-2-chlorbenzolsulfonamid

1-(3-Chlor-4-ethoxyphenyl)-3,3-dimethylharnstoff
2-Methyl-4-chlorphenoxy-thioessigsäureethylester
2-Chlor-3,5-diiod-4-acetoxy-pyridin
1-(4-[2-(4-Methylphenyl)-ethoxy]-phenyl)-3-methyl-3-methoxyharnstoff
2,6-Dimethyl-N-(pyrazolyl-methylenoxymethyl)-2-chloracetanilid
2-Methyl-6-ethyl-N-(pyrazolyl-methylenoxymethyl)-2-chloracetanilid
alpha-2,4-Dichlorphenoxy-propionsäure)-(3-methoxycarbonylamino)-anilid
1-(alpha-2-Brom-4-chlorphenoxypropionsäure)-3-(O-methylcarbamoyl)-anilid
2-Methyl-6-ethyl-N-(pyrazolyl-ethylenoxymethyl)-2-chloracetanilid

2-(3-Trifluormethylphenyl)-4H-3,1-benzoxazin-4-on
2-(3-Pentafluorethoxyphenyl)-4H-3,1-benzoxazin-4-on
2-(3-Trifluormethylthio-phenyl)-4H-3,1-benzoxazin-4-on
2-(3-Difluorchlormethoxyphenyl)-4H-3,1-benzoxazin-4-on

5-Nitro-2-(3-trifluormethyl-phenyl)-4H-3,1-benzoxazin-4-on
5-Chlor-2-(3-trifluormethoxyphenyl)-4H-3,1-benzoxazin-4-on
5-Chlor-2-(3-alpha,alpha,beta,beta-tetrafluorethoxyphenyl)-4H-3,1-benzoxazin-4-on
5-Fluor-2-(3-alpha,alpha,beta,beta-tetrafluorethoxyphenyl)-4H-3,1-benzoxazin-4-on
5-Chlor-2-(4-difluorchlormethoxyphenyl)-4H-3,1-benzoxazin-4-on
5-Fluor-2-(4-difluorchlormethoxyphenyl)-4H-3,1-benzoxazin-4-on
5-Fluor-2-phenyl-4H-3,1-benzoxazin-4-on
5-Fluor-2-(3-difluormethoxyphenyl)-4H-3,1-benzoxazin-4-on
5-Chlor-2-phenyl-4H-3,1-benzoxazin-4-on

N-3-Chlor-4-isopropylphenyl-thiolcarbaminsäuremethylester
6-Methyl-3-methoxy-5,6-dihydro-1,2,4,6-thiatriazin-5-on-1,1-dioxid Natriumsalz
6-Methyl-3-ethoxy-5,6-dihydro-1,2,4,6-thiatriazin-5-on-1,1-dioxid
5-Amino-4-chlor-2-phenyl-3(2H)-pyridazinon
5-Amino-4-brom-2-phenyl-3(2H)-pyridazinon
5-Methylamino-4-chlor-2-(3-trifluormethylphenyl)-3(2H)-pyridazinon
5-Methylamino-4-chlor-2-(3-alpha,alpha,beta,beta-tetrafluorethoxyphenyl)-3(2H)-pyridazinon

5-Dimethylamino-4-chlor-2-phenyl-3(2H)-pyridazinon
4,5-Dimethoxy-2-phenyl-3(2H)-pyridazinon

1-[3'-(2"-Chlor-4"-trifluormethylphenoxy)]-phenyl-4,5-dimethoxy-pyridazinon-6
1-[4'-(3"-Trifluormethyl-phenoxy)]-phenyl-4,5-dimethoxy-pyridazinon-6
N-[4-(4'-Methoxy-phenoxy)-3-chlor-phenyl]-carbaminsäuremethylester
N-[4-(4'-Difluormethoxy-phenoxy)-3-chlor-phenyl]-thio-carbaminsäuremethylester
N-[4-(4'-Difluormethoxy-phenoxy)-phenyl]thio-carbaminsäuremethylester
1-[4-(4'-Methylphenylpropyl)-phenyl]-3-methyl-3-methoxyharnstoff
1-[3-(4'-Chlorphenyl-propyl)-phenyl]-3-methyl-3-methoxyharnstoff
1-[4-(3-Phenyl-2-methyl-propyl)-phenyl]-3-methyl-3-methoxyharnstoff
1-[4-(3-(4'-Chlorphenyl)-2-methyl-propyl)-phenyl]-3-methyl-3-methoxyharnstoff
1-[4-(3-(4'-Methylphenyl)-2-methylpropyl)-phenyl]-3-methyl-3-methoxyharnstoff
2-[1-(N-Ethyloxyamino)-butyliden]-5(4-ethylphenyl)-3-hydroxy-cyclohexen-(2)-on-(1) (Salze)
2-[1-(N-Ethyloxyamino)-butyliden]-5(4-fluorphenyl)-3-hydroxy-cyclohexen-(2)-on-(1) (Salze)
2-[1-(N-Ethyloxyamino)-butyliden]-5-(4-chlorphenyl)-3-hydroxy-cyclohexen-(2)-on-(1) (Salze)
2'-(2,4,6-Trichlorphenyl)-hydrazino-2-cyanacrylsäuremethylester
2-[1-(N-Ethyloxamino)-butyliden]-5-(1,3,3-trimethyl-cyclohexen-1-yl-2)-3--hydroxy-cyclohexen-(2)-on-(1) (Salze)
2-[1-(N-Ethyloxamino)-butyliden]-5-(2,4,4-trimethyl-cyclohexen-1-yl-3)-3--hydroxy-cyclohexen-(2)-on-(1) (Salze)

2-[1-(N-3-Chlorallyl-oxamino)-butyliden]-5-(1-methyl-cyclohex-1-en-4-yl)--3-hydroxy-cyclohexen-(2)-on-(1) (Salze)
3-Isobutoxy-5-methyl-4-methoxycarbonyl-pyrazol
5-Amino-1-(2,4,6-trichlorphenyl)-4-cyano-pyrazol
5-Amino-1-(2,4,6-tribromphenyl)-4-cyano-pyrazol
5-Amino-1-(2,4,6-trichlorphenyl)-4-methoxycarbonyl-pyrazol
5-Amino-1-(2,4-dichlor-6-bromphenyl)-4-methoxycarbonyl-pyrazol
5-Amino-1-(2,6-dichlor-4-bromphenyl)-4-methoxycarbonyl-pyrazol

5-Chlor-2-(3-trifluormethyl-phenyl)-4H-3,1-benzoxazin-4-on
5-Fluor-2-(3-trifluormethyl-phenyl)-4H-3,1-benzoxazin-4-on
2-(3-Tetrafluorethoxyphenyl)-4H-3,1-benzoxazin-4-on
5-Chlor-2-(4'-fluorphenyl)-4H-3,1-benzoxazin-4-on
5-Fluor-2-(4'-fluorphenyl)-4H-3,1-benzoxazin-4-on
5-Fluor-2-(3'fluorphenyl)-4H-3,1-benzoxazin-4-on
5-Chlor-2-(3'-fluorphenyl)-4H-3,1-benzoxazin-4-on
5-Chlor-2-(3'-difluorchlormethylphenyl)-4H-3,1-benzoxazin-4-on
5-Fluor-2-(3'-difluorchlormethylphenyl)-4H-3,1-benzoxazin-4-on

6-Methyl-3-methoxy-5-(4'-nitrophenoxy)-6H-1,2,4,6-thiatriazin-1,1-dioxid
6-Methyl-3-methoxy-5-(propargyloxy-6H-1,2,4,6-thiatriazin-1,1-dioxid
6-Methyl-3-methoxy-5-(2,4-dichlorbenzoxy)-6H-1,2,4,6-thiatriazin-1,1-dioxid

2-(2',4'-Dichlorphenoxy)-2-fluorpropionsäure (Salze, Ester)
2-[4-(5'-Trifluormethylpyridyl-2-oxy)-phenoxy]-propionsäurebutylester
2-[4-(3'-Chlor-5'-trifluormethylpyridyl-2-oxy)-phenoxy]-propionsäure (Salze, Ester)

2-[4-(6-Chlorchinoxalyl-2-oxy)-phenoxy]-propionsäure-pentylester
2-[4-(6-Chlor-chinoxalyl-2-oxy)-phenoxy]-propionsäuremethylester
2-[4-(6-Chlorbenzthiazolyl-2-oxy)-phenoxy]-propionsäure (Salze, Ester)
2-[4-(6-Chlorbenzoxazolyl-2-oxy)-phenoxy]-propionsäure (Salze, Ester)
1-[5-(3-Fluorbenzylthio)-thiadiazolyl-2]-1-methylharnstoff
2-Methoxycarbonyl-N-(3,5-dimethylpyrimidinyl-2-aminocarbonyl)-benzolsulfonamid
alpha-(3,5,6-Trichlor-pyrid-2-yl-oxy)-essigsäure (Salze, Ester)
alpha-(4-Amino-3,5-dichlor-6-fluor-pyrid-2-yl-oxy)-essigsäure (Salze, Ester)
S-[N-(4-Chlorphenyl)-N-isopropyl-carbamoyl-methyl]-O,O-dimethyl-dithiophosphat
Ammonium-(3-amino-3-carboxy-propyl)-methylphosphinat
(Hydroxy)-(methyl)-phosphinyl-L-alpha-aminobutyryl-L-alanyl-Natriumsalz
4-Trifluormethyl-diphenylether
2-(3,5-Dichlorphenyl)-2-(2'2'2'-trichlorethyl)-oxiran
2,4-Diamino-5-methylthio-6-chlor-pyrimidin

N-(4-Ethylthio-2-trifluormethyl-phenyl)-methylsulfonamid

3-Methoxy-4-methyl-5(3-methyl-2-butenyloxy)-1,2-di(hydroxymethyl)-benzol

2-(3,5-Dimethylphenoxy)-2-(1,2,4-triazolyl-1)-essigsäure-N-tertiär-butylamid

2-(3,5-Dichlorphenoxy)-2-(1,2,4-triazolyl-1)-essigsäure-N-tertiär-butylamid

3,7-Dichlor-8-chinolincarbonsäure (Salze, Ester)

5-(2-Chlor-4-trifluormethyl-phenoxy)-2-nitro-N-(1-methoxycarbonyl-ethoxy)-benzamid

N-[3-(1-Ethyl-1-methylpropyl)-isoxazolyl-5]-2,6-dimethoxybenzamid

2'-Methoxyethyl-2-[5-(2-chlor-4-trifluormethyl-phenoxy)-2-nitrophenoxy]-propionat

Methyl-6-(4-isopropyl-4-methyl-5-oxo-2-imidazolin-2-yl)-3-methylbenzoat

Methyl-6-(4-isopropyl-4-methyl-5-oxo-2-imidazolin-2-yl)-4-methylbenzoat

Benzyltrimethylammoniumchlorid

1-[alpha-(4-Trifluormethyl-phenoxy)-phenoxy-propionsäure]-3-(O-methylcarbamoyl)-anilid

1-Dodecyl-cycloheptan-2-on

N-[2-Chlor-4-methylsulfonyl-phenyl]-chlormethansulfonamid

N-[2-Brom-4-ethylsulfonyl-phenyl]-chlormethansulfonamid

N-[2,3-Dichlor-4-ethylsulfonyl-phenyl]-chlormethansulfonamid

2-[1-(N-Ethoxyamino)-pyropyliden-]-5-(pyrid-3-yl)-3-hydroxy-cyclohex-2-en-1-on (Salze)

2,4,5-Trichlorphenoxyessigsäure (Salze, Ester, Amide)

2-[1-(N-Ethoxyamino)-butyliden]-5-(tetrahydropyran-3-yl)-3-hydroxy-cyclohex-2-en-1-on (Salze)

2-[1-(N-Ethoxyamino)-butyliden]-5-(4-methyl-tetrahydropyran-3-yl)-3-hydroxy-cyclohex-2-en-1-on (Salze)

2-[1-(N-Ethoxyamino)-butyliden]-5-(tetrahydrothiopyran-3-yl)-3-hydroxy-cyclohex-2-en-1-on (Salze)

2-[1-(N-Ethoxyamino)-propyliden]-5-(pyrid-3-yl)-3-hydroxy-cyclohex-2-en-1-on (Salze)

2-[1-(N-Allyloxamino)-propyliden]-5-(pyrid-3-yl)-3-hydroxy-cyclohex-2-en-1-on

2-[1-(N-Ethoxyamino)-butyliden]-5-(pyrid-3-yl)-3-hydroxy-cyclohex-2-en--1-on (Salze)

2-[1-(N-Allyloxyamino)-butyliden]-5-(pyrid-3-yl)-3-hydroxy-cyclohex-2-en--1-on (Salze)

2-[4,5-Dihydro-4-methyl-4-isopropyl-5-oxo-1H-imidazol-2-yl]-3-chinolin-carbonsäure

2-[4,5-Dihydro-4-methyl-4-isopropyl-5-oxo-1H-imidazol-2-yl]-nicotinsäure-isopropylaminsalz

2-Chlor-2'-methyl-6'-ethyl-N-(N'-1-methoxycarbonyl)-ureidomethylacet-anilid

2-Chlor-2'-6'-diethyl-N-(N'-1-methoxycarbonyl)-ureidomethylacetanilid

2-Chlor-2'-6'-dimethyl-N-(N'-1-methoxycarbonyl)-ureidomethylacetanilid

2-Chlor-6-nitro-3-phenoxy-anilin

N-Phosphonomethyl-glycin-trimethyl-sulfoniumsalz

5-(2-Chlor-4-trifluormethyl-phenoxy)-2-nitro-N-methansulfonyl-benzamid

5-(2-Chlor-4-trifluormethyl-phenoxy)-2-nitro-benzoesäure-1-ethoxycarbonyl--ethyl)-ester

1-[3'-(2"-Chlor-4"-trifluormethyl-phenyl-thio)-phenyl]-4,5-dimethoxy--pyridazon-(6)

3-Methyl-6-fluor-5H-thiazolo[2,3-b]-chinazolin-5-on

3-Methyl-2-sulfosäure-5H-thiazolo[2,3-b]-chinazolin-5-on

3-Methyl-2-brom-5H-thiazolo[2,3-b]-chinazolin-5-on

5H-Triazolo[2,3-b]-chinazolin-5-on

2-(1-Ethoxyamino-butyliden)-5-cyclododeca-1,5-dion-9-yl-cyclohex-1-en--1-on

2-(1-Ethoxyamino-butyliden)-5-cyclododecyl-cyclohex-1-en-1-on

5-(4'-Trifluormethyl-2'-chlor-phenoxy)-2-nitro-benzylthioessigsäure--(2-trimethylsilylethyl)-ester

2-(2-Chlorbenzyl)-4,4-dimethyl-isoxazolidin-3-on

N-[4-(3,4-Dichlorbenzyloxymethyl)-phenyl]-N'-methyl-N'-methoxy-harnstoff

N-[4-(4-Trifluormethylbenzyloxymethyl)-phenyl]-N'-methyl-N'-methoxy-harnstoff

N-[3-Chlor-4-(1-benzyloxy-ethyl)-phenyl]-N'-methyl-N'-methoxy-harnstoff

N-[4-(4-Trifluormethyl-benzyloxymethyl)-phenyl]-N',N'-dimethyl-harnstoff

3-(2'-Chlor-4'-trifluormethylphenoxy)-6-nitro-benzensäure-N-methyl--sulfenamid

3-(2'-Chlor-4'-trifluormethylphenoxy)-6-nitro-benzensulfensäureisopropylester

1-Methyl-4-isopropyl-2-(2-methylbenzyloxy)-exo-7-oxabicyclo-[2.2.1]heptan

5-(2-Chlor-4-trifluormethylphenoxy)-2-nitro-acetophenonoxim-O-essigsäuremethylester

O-(3-Phenyl-6-chlorpyridazin-4-yl)-S-n-octyl-thiolcarbonat

3-Methyl-7-chlor-chinolin-8-carbonsäure (Salze, Ester)

3-Ethyl-7-chlor-chinolin-8-carbonsäure (Salze, Ester)

2,6-Diethyl-N-(but-2-inyl)-2-chloracetanilid

2-Chlor-4-trifluormethyl-3'-[(3"-carboxy-propionyl)-hydrazino]-4'-nitro-diphenylether (Natriumsalz)

N-[(4-Chlor-6-methoxy-pyrimidin-2-yl)-aminocarbonyl]-2-ethoxycarbonyl-benzolsulfonamid

N-[(4-Methoxy-6-methyl-1,3,5-triazin-2-yl)-aminocarbonyl]-2-methoxycarbonyl-benzolsulfonamid

2-[1-(N-Ethoxyamino)-butyliden]-5-(4a,7,8,8a-tetrahydro-2H,5H-pyrano[4,3-b]-pyran-3-yl)-3-hydroxy-cyclohexen-2-en-1-on

2-[1-(N-Allyloxamino)-butyliden]-5-(4a,7,8,8a-tetrahydro-2H,5H-pyrano[4,3-b]-pyran-3-yl)-3-hydroxy-cyclohexen-2-en-1-on

2-[1-(N-Allyloxamino)-butyliden]-5-(3,4,4a,7,8,8a-hexahydro-2H,5H-pyrano[4,3-b]-pyran-3-yl)-3-hydroxy-cyclohexen-2-en-1-on

2-[1-(N-Ethoxyamino)-butyliden]-5-(3,4,4a,7,8,8a-hexahydro-2H,5H-pyrano[4,3-b]-pyran-3-yl)-3-hydroxy-cyclohexen-2-en-1-on

2-[4,5-Dihydro-4-methyl-4-isopropyl-5-oxo-1H-imidazol-2-yl]-5-ethyl-pyridin-3-carbonsäure

2-(1-Ethoxyamino-butyliden)-5-(5,6-dihydro-2H-1,1-dioxothiopyran-3-yl)-3-hydroxy-cyclohex-2-en-1-on

2-(1-Ethoxyamino-propyliden)-5-(5,6-dihydro-2H-1,1-dioxothiopyran-3-yl)-3-hydroxy-cyclohex-2-en-1-on

2-(1-Propargylamino-butyliden)-5-(5,6-dihydro-2H-1,1-dioxothiopyran-3-yl)-3-hydroxy-cyclohex-2-en-1-on

3-(2'-Chlor-4'-trifluormethylphenoxy)-6-nitro-phenylglyoxylsäuremethylester

2-[4-(2-Chlor-4-trifluormethylphenoxy)-phenoxy]-propionsäurebutylester

2-Carboxy-N-[[(4-methoxy-6-chlorpyrimidin-2-yl)-amino]-carbonyl]-benzolsulfonamid

Außerdem kann es von Nutzen sein, die Verbindungen der Formel I allein oder in Kombination mit anderen Herbiziden, auch noch mit weiteren Pflanzenschutzmitteln gemischt gemeinsam auszubringen, beispielsweise mit Mitteln zur Bekämpfung von Schädlingen oder phytopathogenen Pilzen bzw. Baktgerien. Von Interesse ist ferner die Mischbarkeit mit Mineralsalzlösungen, welche zur Bekämpfung von Ernährungs- und Spurenelementmängeln eingesetzt werden.

Patentansprüche

1. 4H-Pyrido[2,3-d][1,3]oxazin-4-on-derivate der Formel I

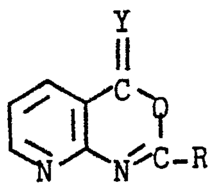

(I),

in der

Y für Sauerstoff oder Schwefel und

R für einen durch Halogen, $C_1$-$C_4$-Halogenalkyl, mit Ausnahme von Trifluormethyl, $C_1$-$C_4$-Halogenalkoxy, $C_1$-$C_4$-Halogenalkylmercapto, $C_1$-$C_4$-Halogenalkylsulfinyl oder $C_1$-$C_4$-Halogenalkylsulfonyl o-, m- oder p-substituierten Phenylrest stehen.

2. 4H-Pyrido[2,3-d][1,3]oxazin-4-on-derivate der Formel I gemäß Anspruch 1 dadurch gekennzeichnet, daß Y für Sauerstoff oder Schwefel und

R für einen durch Halogen, $C_1$-$C_4$-Halogenalkyl, mit Ausnahme von Trifluormethyl, oder $C_1$-$C_4$-Halogenalkylmercapto o-, m- oder p-substituierten Phenylrest stehen.

3. 4H-Pyrido[2,3-d][1,3]oxazin-4-on-derivate der Formel I gemäß Anspruch 1, dadurch gekennzeichnet, daß Y Sauerstoff bedeutet.

4. Verfahren zur Herstellung von 4H-Pyrido[2,3-d][1,3]oxazin-4-on-derivaten der Formel I gemäß Anspruch 1, dadurch gekennzeichnet, daß man eine Säure der Formel II

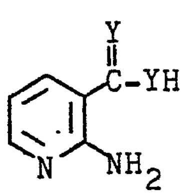

(II)

in der Y die im Anspruch 1 genannten Bedeutungen hat, mit mindestens dem zweifachen molaren Überschuß eines Carbonsäurehalogenids der Formel III

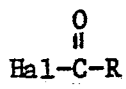
(III),

in der
R die im Anspruch 1 genannten Bedeutungen hat und Hal für Halogen steht, in einem aromatischen tertiären Amin als Lösungsmittel bei einer Temperatur im Bereich zwischen 10 und 90°C umsetzt.

5. Verfahren zur Herstellung von 4H-Pyrido[2,3-d][1,3]oxazin-4-on-derivaten der Formel I gemäß Anspruch 1, dadurch gekennzeichnet, daß man eine Säure der Formel II

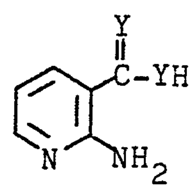
(II),

in der Y die im Anspruch 1 genannten Bedeutungen hat, oder ein Alkalimetall- oder Erdalkalimetallsalz dieser Aminonicotinsäure mit ungefähr stöchiometrischen Mengen eines Carbonsäurehalogenids der Formel III

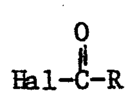
(III),

in der R die im Anspruch 1 genannten Bedeutungen hat und Hal für Halogen steht, in einem inerten organischen Lösungsmittel oder in Wasser und gegebenenfalls in Anwesenheit eines Säureacceptors bei einer Temperatur im Bereich von 0 bis 90°C zu einem Carbonamid der Formel IV

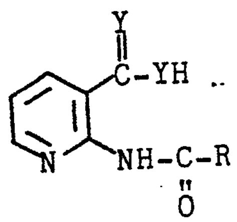

(IV),

in der R und Y die im Anspruch 1 genannten Bedeutungen haben, umsetzt und dieses dann in Gegenwart eines wasserentziehenden Mittels bei einer Temperatur im Bereich zwischen 30 und 150°C cyclisiert.

6. Herbizid, enthaltend ein 4H-Pyrido[2,3-d][1,3]oxazin-4-on-derivat der Formel Ia

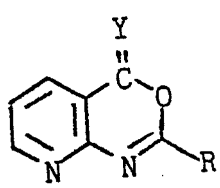

(Ia),

in der
Y für Sauerstoff oder Schwefel und

R für einen gegebenenfalls durch Halogen, $C_1$-$C_4$-Halogenalkyl, $C_1$-$C_4$-Halogenalkoxy oder $C_1$-$C_4$-Halogenalkylmercapto, $C_1$-$C_4$-Halogenalkylsulfinyl oder $C_1$-$C_4$-Halogenalkylsulfonyl o-, m- oder p-substituierten Phenylrest stehen.

7. Herbizid, enthaltend ein 4H-Pyrido[2,3-d][1,3]oxazin-4-on-derivat der Formel Ia gemäß Anspruch 6, dadurch gekennzeichnet, daß Y für Sauerstoff oder Schwefel und R für einen gegebenenfalls durch Halogen, $C_1$-$C_4$-Halogenalkyl, $C_1$-$C_4$-Halogenalkoxy oder $C_1$-$C_4$-Halogenalkylmercapto o-, m- oder p-substituierten Phenylrest stehen.

8. Herbizid, enthaltend ein 4H-Pyrido[2,3-d][1,3]oxazin-4-on-derivat der Formel Ia gemäß Anspruch 6, dadurch gekennzeichnet, daß Y für Sauerstoff steht.

9. Verfahren zur Bekämpfung unerwünschten Pflanzenwuchses, dadurch gekennzeichnet, daß man die Pflanzen oder den Boden mit einer herbizid wirksamen Menge eines 4H-Pyrido[2,3-d][1,3]oxazin-4-on-derivats der Formel Ia gemäß Anspruch 6 behandelt.

10. Verwendung eines 4H-Pyrido[2,3][1,3]oxazin-4-on-derivats der Formel Ia

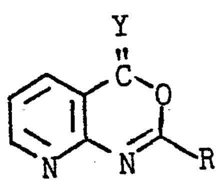

(Ia),

in der

Y für Sauerstoff oder Schwefel und

R für einen gegebenenfalls durch Halogen, $C_1$-$C_4$-Halogenalkyl, $C_1$-$C_4$-Halogenalkoxy, $C_1$-$C_4$-Halogenalkylmercapto, $C_1$-$C_4$-Halogenalkylsulfinyl oder $C_1$-$C_4$-Halogenalkylsulfonyl o-, m- oder p-substituierten Phenylrest stehen, als herbizides Mittel.